# EUROPEAN PATENT SPECIFICATION

(11) **EP 3 965 695 B1**
(45) Date of publication and mention of the grant of the patent: **15.04.2026**
(21) Application number: 20801523.0
(22) Date of filing: 08.05.2020
(51) Int. Cl.: A61F 2/24, A61F 2/00, A61F 2/02, A61F 2/95, A61F 2/962

(54) **ANCHOR CONFIGURATIONS FOR PROSTHETIC HEART VALVES**
ANKERKONFIGURATIONEN FÜR HERZKLAPPENPROTHESEN
CONFIGURATIONS D'ANCRAGES DE PROTHÈSES DE VALVULE CARDIAQUE

(30) Priority: 09.05.2019 US 201962845856 P
(43) Date of publication of application: 16.03.2022
(73) Proprietor: Caisson Interventional, LLC, Maple Grove, Minnesota 55311 (US)
(72) Inventor: ASKGAARD, Gunnar, Maple Grove, Minnesota 55311 (US); SCHNEIDER, Lucas, Champlin, Mississippi 55316 (US); MONTAG, Benjamin, Delano, Minnesota 55328 (US); BAHOORA, Kim, Maple Grove, Minnesota 55311 (US); RAMASWAMY, Vidhya, Maple Grove, Minnesota 55311 (US); POCRNICH, John, Maple Grove, Minnesota 55311 (US); MORTIER, Todd, Mound, Minnesota 55364 (US); SCHWEICH, Cyril, Maple Grove, Minnesota 55311 (US); GANESAN, Kavitha, Minnetrista, Minnesota 55364 (US); IYER, Ramji, Maple Grove, Minnesota 55311 (US)
(74) Representative: Grünecker Patent- und Rechtsanwälte PartG mbB
(86) International application number: PCT/US2020/032235
(87) International publication number: WO 2020/227686

(56) References cited:
- WO-A1-2013/059747
- WO-A2-2012/177942
- US-A1- 2015 359 629
- US-A1- 2016 113 768
- US-A1- 2016 270 911
- US-A1- 2017 128 204
- US-A1- 2017 189 177
- US-A1- 2017 231 759
- US-A1- 2018 049 873
- US-B2- 10 010 414

## Description

### TECHNICAL FIELD

The present disclosure relates to heart valve interventional systems and methods and, more particularly, to mitral heart valve therapy systems and methods.

### BACKGROUND

Human heart valves, which include the aortic, pulmonary, mitral and tricuspid valves, function in synchronization with the pumping heart to control the flow of blood between chambers of the heart. In short, the valves allow blood to flow downstream and inhibit blood from flowing upstream. Diseased heart valves exhibit impairments such as narrowing of the valve, remodeling of the annulus, or calcification, which inhibit the valves from properly controlling blood flow. Such impairments reduce the heart's blood-pumping efficiency and can be a debilitating or, in some situations, life threatening. Thus, extensive efforts have been made to develop methods and devices to repair or replace impaired heart valves.

One technique for addressing a damaged or defective heart valve is to replace the native valve with a valve prosthesis. One category of heart valve prosthesis includes those that can be delivered in a minimally invasive fashion so as to minimize trauma to the patient. Replacement valves are being designed to be delivered through minimally invasive procedures and even percutaneous procedures. Such replacement valves often include a tissue-based valve that is connected to an expandable frame that is then delivered to the native valve's annulus.

Development of prostheses including but not limited to replacement heart valves that can be collapsed for minimally invasive delivery and then controllably expanded has proven to be particularly challenging. An additional challenge relates to the ability of such prostheses to be secured relative to adjacent native tissue. Adequate anchoring of the prosthesis is important to ensuring the successful operation of the prosthetic heart valve for a sufficient length of time.

WO 2012/177942 discloses prosthetic heart valve devices for percutaneous replacement of native valves, featuring an expandable support and asymmetrically arranged arms configured to engage native valve leaflets and subannular surfaces. The invention provides configurations allowing stable anchoring and controlled leaflet engagement, suitable for mitral, aortic, or tricuspid valve applications.

### SUMMARY

The present disclosure describes shapes and geometries of anchoring elements, including feet, used to secure a prosthetic valve, e.g., a prosthetic mitral valve, in the native heart valve annular position. The invention relates to a device as defined by claim 1 and the method of manufacturing as defined by claim 5. In embodiments, the foot geometry/directionality is designed such that the foot loads to the fibrous annular region that is made of collagenous tissue with higher puncture resistance. Additionally, the foot contact surface area is designed such that the pressure exerted by the foot is below the pressure required to puncture the heart tissue, such as the left ventricular muscle wall tissue.

### BRIEF DESCRIPTION OF THE DRAWINGS

Fig. 1A is a diagram illustrating a top (atrial) view of a heart valve prosthesis configured to be deployed within a native heart valve at a native heart valve annulus, in accordance with embodiments of the subject matter of the disclosure.
Fig. 1B is a diagram illustrating an anterior view of the heart valve prosthesis, in accordance with embodiments of the subject matter of the disclosure.
Fig. 2 is a diagram illustrating a prosthetic mitral valve annulus and anchor locations for the feet of the anchors disposed about the circumference of the prosthetic mitral valve annulus, in accordance with embodiments of the subject matter of the disclosure.
Fig. 3A is a schematic diagram illustrating a mitral valve having an annulus, a transition region below the annulus, and LV muscle below the transition region.
Fig 3B is a diagram illustrating tissue at the mitral valve, including the annulus, the transition region below the annulus, and the LV muscle situated below the transition region.
Fig. 4 is a diagram illustrating portions of a heart valve prosthesis, in accordance with embodiments of the subject matter of the disclosure.
Fig. 5A is a diagram illustrating a 30 degree foot angle of an anchor and foot with respect to the longitudinal axis of the valve, in accordance with embodiments of the subject matter of the disclosure.
Fig. 5B is a diagram illustrating a 0 degree foot angle of an anchor and foot with respect to the longitudinal axis of the valve, in accordance with embodiments of the subject matter of the disclosure.
Fig. 6 is a diagram illustrating embodiments of the profile of a foot, in accordance with embodiments of the subject matter of the disclosure.
Fig. 7 is a diagram illustrating an anchor and a foot having one of the diamond-like structures as depicted in iterations C-E (shown in Fig. 6), in accordance with embodiments of the subject matter of the disclosure.
Fig. 8 is a diagram illustrating a native mitral valve and multiple anchor locations for the feet of a heart valve prosthesis around the circumference of the native mitral valve, in accordance with embodiments of the subject matter of the disclosure.

### DETAILED DESCRIPTION

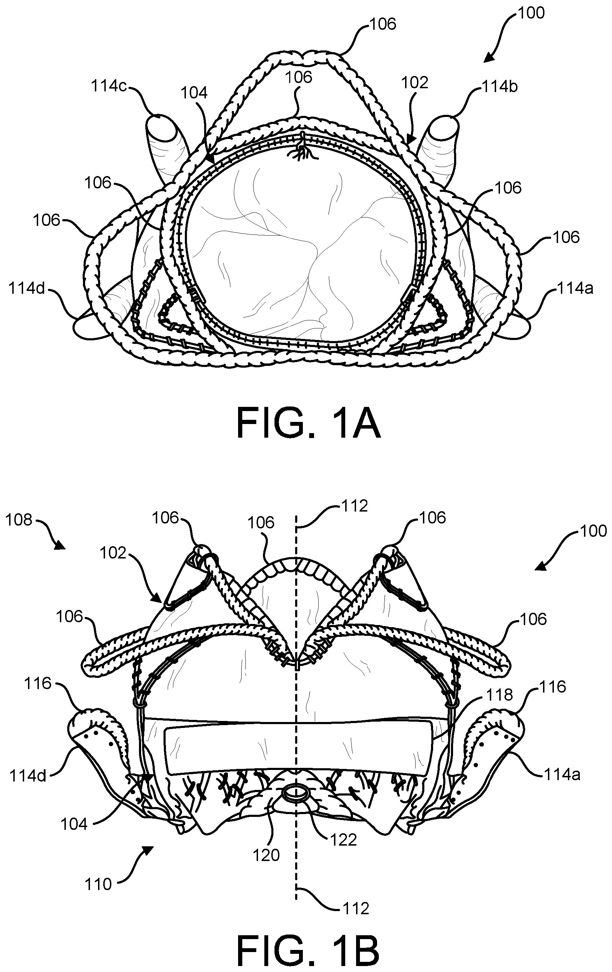

Fig. 1A is a diagram illustrating a top (atrial) view of a heart valve prosthesis 100 configured to be deployed within a native heart valve at a native heart valve annulus, in accordance with embodiments of the subject matter of the disclosure. Fig. 1B is a diagram illustrating an anterior view of the heart valve prosthesis 100, in accordance with embodiments of the subject matter of the disclosure. Figs. 1A and 1B illustrate the heart valve prosthesis 100 in an expanded configuration, as opposed to a collapsed configuration that is used for delivery of the heart valve prosthesis 100 to the native heart valve annulus.

The prosthesis 100 includes an anchor assembly 102 and a valve assembly 104. In some embodiments, the occluding function of the prosthesis 100 can be performed using configurations other than the depicted tri-leaflet occluder. For example, bi-leaflet, quad-leaflet, or mechanical valve constructs can be used in some embodiments.

The anchor assembly 102 includes an expandable frame 106 having a proximal end 108 and a distal end 110 with a longitudinal axis 112 extending therethrough. The expandable frame 106 is configured to collapse radially for delivery and expand radially upon deployment to the expanded configuration.

The anchor assembly 102 includes a plurality of spaced anchors 114a-114d extending from the distal end 110 of the expandable frame 106 towards the proximal end 108. Each of the anchors 114a-114d includes a free end or foot 116. Figs. 1A and 1B illustrate the anchors 114a-114d in an expanded anchor configuration for engaging subannular tissue (below the native heart valve annulus). Also, each of the anchors 114a-114d is expandable from a collapsed anchor configuration where the anchors 114a-114d are inverted such that the anchors 114a-114d point distally or downward in the collapsed anchor configuration.

As shown in Fig. 1B, a supplemental covering portion 118 can be positioned on an anterior surface of the valve assembly 104. The supplemental covering portion 118 can provide an enhanced sealing capability between the valve prosthesis 100 and surrounding native tissues. The supplemental covering portion 118 can be made of a material such as, but not limited to, DACRON^{®}, felt, polyester, a silicone, a urethane, ELAST-EON^{™} (a silicone and urethane polymer), another biocompatible polymer, polyethylene terephthalate (PET), copolymers, or combinations and subcombinations thereof. In embodiments, the valve prosthesis 100 also includes a systolic anterior motion (SAM) containment member 120 with an eyelet 122 for engaging and moving the SAM containment member 120.

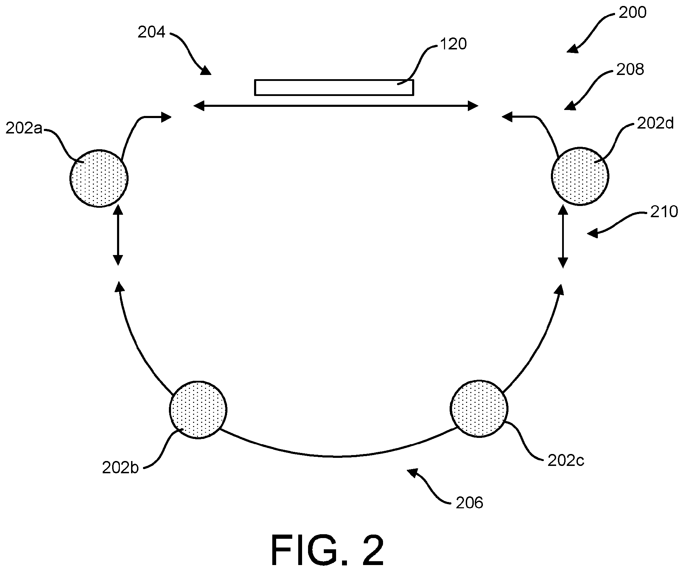

Fig. 2 is a diagram illustrating a prosthetic mitral valve annulus 200 and anchor locations 202a-202d for the feet 116 of the anchors 114a-114d disposed about the circumference of the prosthetic mitral valve annulus 200, in accordance with embodiments of the subject matter of the disclosure. The prosthetic mitral valve annulus 200 includes an anterior region 204, a posterior region 206, a commissural region 208, and a medial/lateral region 210. In embodiments, the prosthetic mitral valve 100 includes a SAM containment member 120 at the anterior region 204.

The heart valve prosthesis 100 includes four anchors 114a-114d, such that two of the anchors 114a and 114d are generally disposed at the anchor locations 202a and 202d, respectively, in the anterior region 204 and two of the anchors 114b and 114c are generally disposed at the anchor locations 202b and 202c, respectively, in the posterior region 206. In embodiments, the heart valve prosthesis 100 can include three anchors, two of which are generally disposed in the anterior region 204 and one of which is disposed in a generally central location of the posterior region 206. In other embodiments, the heart valve prosthesis 100 can include more than three or four anchors.

In some embodiments, the heart valve prosthesis 100 design and configuration are any one of the prosthesis designs and configurations disclosed in United States Patent Application Publication No. 2017/0189177 or United States Patent Application Publication No. 2019/0029814. While the concepts disclosed herein may be used in conjunction with any heart valve, the following disclosure provides embodiments for a mitral valve prosthesis.

While the anchor locations 202a-202d are illustrated in certain locations around the circumference of the prosthetic mitral valve annulus 200 in Fig. 2, in embodiments, one or more of these locations can be adjusted, such as up to 10 degrees (clockwise or counterclockwise), about the circumference, i.e., perimeter, of the annulus 200. In some embodiments, the anchor locations 202a-202d are disposed at a circumferential location about the annulus 200, such that the anchor locations 202a-202d are generally aligned with native valve commissures. In this way, the interference of the anchors 114a-114d with the operation of the native leaflets is minimized.

By disposing the anchors 114a-114d at appropriate locations about the circumference of the annulus 200, the heart valve prosthesis 100 is adequately anchored such that during diastole, when the left ventricle contracts and the blood pressure drives the valve prosthesis toward the left atrium, the anchors 114a-114d contact subannular tissue, i.e., tissue below the annulus of the native heart valve, and thereby anchor the prosthesis 100 at the mitral valve annulus location.

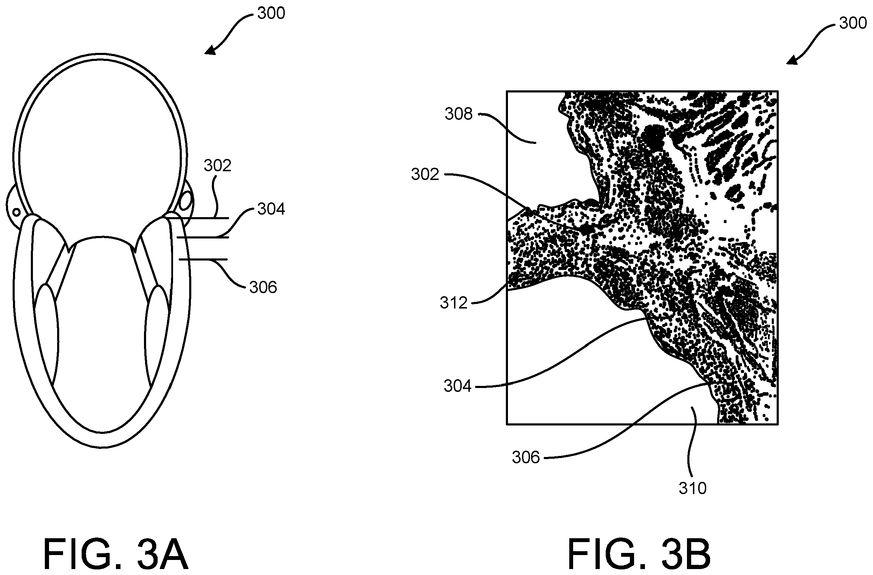

Fig. 3A is a schematic diagram illustrating a mitral valve 300 having an annulus 302, a transition region 304 below the annulus 302, and LV muscle 306 below the transition region 304. The location and size of these regions or areas may vary slightly from patient to patient. For example, in embodiments, the transition region 304 begins 1 to 3 millimeters (mm) below the annulus 302 and the LV muscle 306 begins 6 to 8 mm below the annulus 302, and, in embodiments, the transition region 304 ends where the LV muscle 306 begins. In embodiments, the transition region 304 is about 2 millimeters (mm) below the annulus 302 and the LV muscle 306 is about 7 mm below the annulus 302.

Fig 3B is a diagram illustrating tissue at the mitral valve 300, including the annulus 302, the transition region 304 below the annulus 302, and the LV muscle 306 situated below the transition region 304. As shown, the annulus 302 is situated between the left atrium 308 and the left ventricle 310, and a leaflet 312 branches from the annulus 302.

The annulus 302 is made up of fibrous tissue, such as collagen and/or reticular fibers, which have significantly high puncture resistance. The LV muscle substrate 306 is made up of cardiac muscle cells that have a somewhat lower puncture resistance as compared to the annulus 302. In embodiments, the prosthetic valve anchors 114a-114d load to the tissue of the annulus 302, the LV muscle 306, or the transition region 304.

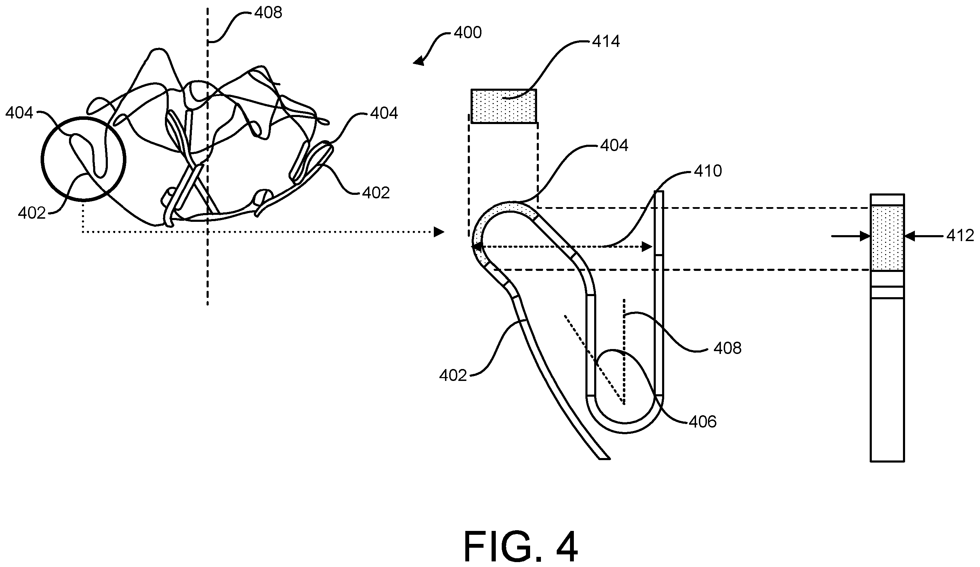

Fig. 4 is a diagram illustrating portions of a heart valve prosthesis 400, in accordance with embodiments of the subject matter of the disclosure. As shown, the prosthesis 400 includes anchors 402 with feet 404 for contacting tissue adjacent the native heart valve. In embodiments, the prosthesis 400 is like the heart valve prosthesis 100 of Figs. 1A and 1B. Also, in embodiments, the anchors 402 and feet 404 are like the anchors 114a-114d and feet 116 (shown in Figs. 1A and 1B).

The anchors 402 and the feet 404 are configured to contact the subannular tissue on the ventricular side of the valve annulus. As shown in Fig. 4, the foot 404 is configured with a "foot angle" 406 defined as the angle of the foot 404 with respect to the longitudinal axis 408 of the heart valve prosthesis 400 and a "toe out distance" 410, which is defined as the distance the foot 404 extends radially outwardly from the valve body. Additionally, the foot 404 includes a certain foot width 412 traveling through a certain arc length, which collectively defines a foot contact surface area, indicated at 414. By adjusting these parameters, the foot contact surface area at 414 may be adjusted to an appropriate level to properly support the forces generated during the heart cycle. For example, by increasing the foot contact surface area at 414, the pressure on the tissue adjacent the annulus may be reduced.

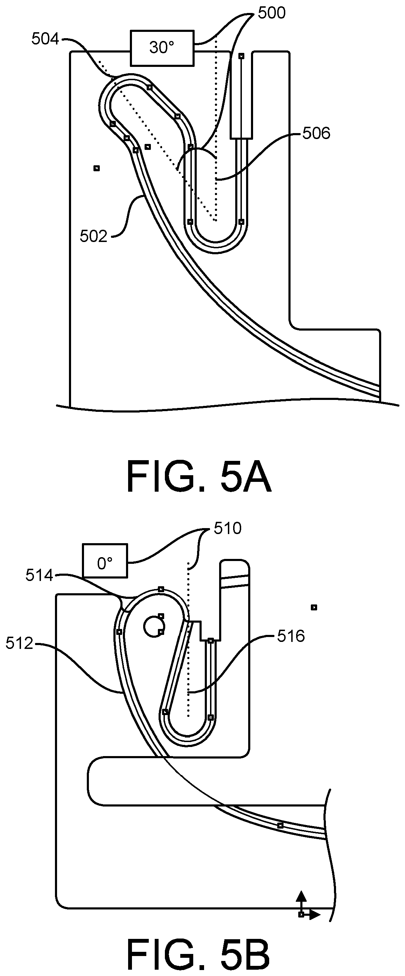

Figs. 5A and 5B are diagrams illustrating various foot angles of anchors and feet, in accordance with embodiments of the subject matter of the disclosure. The foot angles in Figs. 5A and 5B are defined as the angle of the foot with respect to the longitudinal axis of the heart valve prosthesis, as described above.

Fig. 5A is a diagram illustrating a 30 degree foot angle 500 of anchor 502 and foot 504 with respect to the longitudinal axis, indicated at 506, of the valve, in accordance with embodiments of the subject matter of the disclosure.

Fig. 5B is a diagram illustrating a 0 degree foot angle 510 of anchor 512 and foot 514 with respect to the longitudinal axis, indicated at 516, of the valve, in accordance with embodiments of the subject matter of the disclosure. Where, the anchor 512 and foot 514 are substantially parallel to the longitudinal axis 516 of the valve. In embodiments, the foot geometry is configured such that the foot angle is within the range of 0 to 45 degrees with respect to the longitudinal axis of the valve. In embodiments, the 0 degree foot angle 510 aligns the foot 514 with the fibrous annulus tissue of the heart valve.

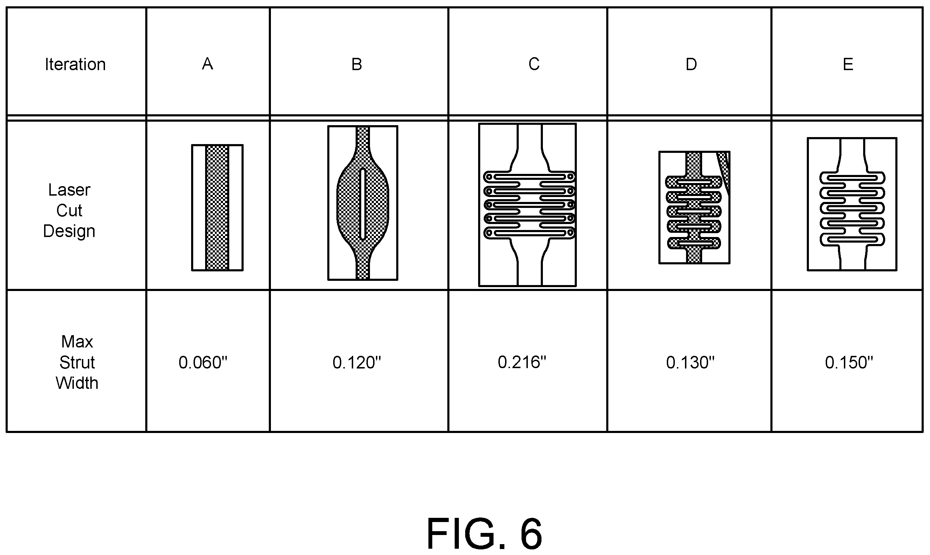

In some embodiments, the foot geometry is designed to ensure that the foot contact surface area 414 is such that the maximum pressure exerted by the foot is less than the puncture resistance of the substrate, i.e., the native heart valve tissue contacted by the foot of the prosthesis. Where, the foot geometry and the foot contact surface area 414 are based on multiple items, such as the foot angle, the arc length of the foot, the arc radius of the foot, and the foot width, which can be adjusted to ensure that the maximum pressure exerted by the foot is less than the puncture resistance of the substrate. In addition, the contact surface area 414 can be adjusted or controlled by modifying the profile of the foot at the contact location, as illustrated in Fig. 6, which may be a laser cut profile.

Fig. 6 is a diagram illustrating embodiments of the profile of a foot, such as the feet 404, 504, and 514, in accordance with embodiments of the subject matter of the disclosure. As illustrated, iteration A of the foot has a straight width with a maximum width of .06 inches, iteration B has a hexagonal shaped, diamond-like structure (hexagonal, diamond-like structure being a requirement of the invention as further disclosed in claim 1) with a maximum width of .12 inches (1 inch= 25.4 mm), iteration C has multiple diamond-like structures with a maximum width of .216 inches, iteration D has multiple diamond-like structures with a maximum width of .13 inches, and iteration E has multiple diamond-like structures with a maximum width of .15 inches.

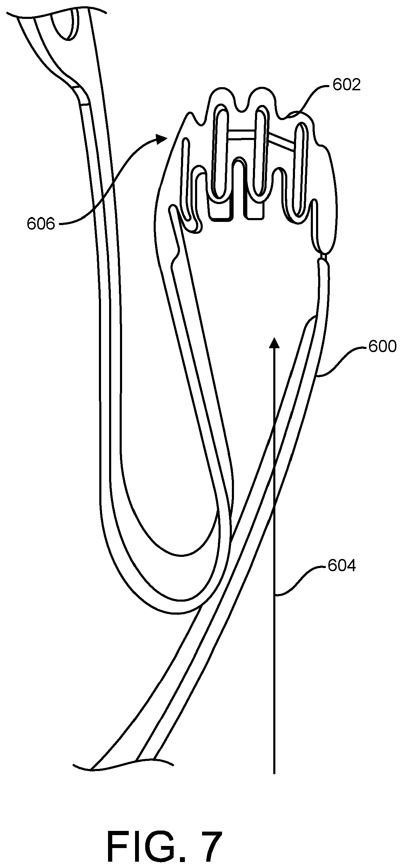

Fig. 7 is a diagram illustrating an anchor 600 and a foot 602 having one of the diamond-like structures as depicted in iterations C-E (shown in Fig. 6), in accordance with embodiments of the subject matter of the disclosure. The anchor 600 and the foot 602 are at a foot angle 604 of 0 degrees and the multiple diamond-like structures on the foot 602 provide an increased loading or contact surface area 606.

Also, addition of the diamond-like structures in iterations B-E, as compared to only increasing the strut width, allows for easier formability and manufacturability of these parts as well as improved deliverability.

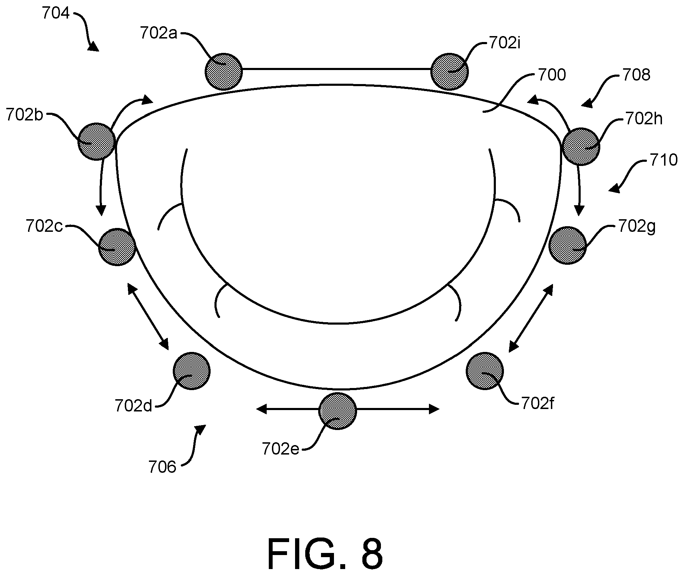

Fig. 8 is a diagram illustrating a native mitral valve 700 and multiple anchor locations 702a-702i for the feet of a heart valve prosthesis around the circumference of the native mitral valve 700, in accordance with embodiments of the subject matter of the disclosure. The native mitral valve 700 includes an anterior region 704, a posterior region 706, a commissural region 708, and a medial/lateral region 710.

By adjusting variables including one or more of the locations of the anchors, the number of anchors, the number of feet, the number of feet per anchor, foot angles, foot widths, arc length, and arc radius, the transfer forces and puncture pressures generated by the prosthetic valve may be increased or decreased. Where, in embodiments, a primary function of the foot is to provide stable anchoring to the native heart valve without puncturing into the loading tissue, i.e., the substrate, of the heart. Also, in embodiments, the optimized foot locations in combination with the foot geometry ensure that the foot does not puncture into or through the substrate.

## Claims

1. A valve prosthesis (100) configured to be deployed within a native heart valve at a native heart valve annulus (200), the valve prosthesis comprising: an expandable frame (106) comprising a proximal end (108) and a distal end (110) and a longitudinal axis (112) extending therethrough, the expandable frame (106) configured to collapse radially for delivery and expand radially upon deployment to an expanded configuration; and a plurality of spaced anchors (114a-114d) extending from the distal end (110) of the frame (106) towards the proximal end (108), each anchor (114a-114d) formed with a foot (116) being a free end portion of the anchor and comprising a contact surface configured for contacting tissue adjacent to the native heart valve, and each anchor (114a-114d) being expandable from a collapsed anchor configuration to an expanded anchor configuration; wherein each of the anchors (114a-114d) includes a foot angle of from 0 to 45 degrees relative to the longitudinal axis (112), wherein the foot angle is defined as the angle between an axis running through the center of the feet (116) and the longitudinal axis (112) of the valve prosthesis (100); and wherein the contact surface of at least one of the plurality of spaced anchors (114a-114d) includes a hexagonal-shaped, diamond-like structure.

2. The valve prosthesis of claim 1 wherein the plurality of spaced anchors are configured as anchoring feet.

3. The valve prosthesis of claim 2 wherein the prosthesis includes two anterior anchors and two posterior anchors.

4. The valve prosthesis of one of the preceding claims, wherein the foot angle of at least one of the spaced anchors relative to the longitudinal axis is 0 degrees, such that the at least one of the spaced anchors is configured to load tissue of the annulus.

5. A method of manufacturing a valve prosthesis (100) configured to be deployed in a native heart valve at a native heart valve annulus (200), the method comprising: forming an expandable frame (106) comprising a proximal end (108) and a distal end (110) and a longitudinal axis (112) extending therethrough, the expandable frame (106) configured to collapse radially for delivery and expand radially upon deployment to an expanded configuration; and forming a plurality of anchors (114a-114d) extending from the distal end (110) of the frame (106) towards the proximal end (108), each anchor (114a-114d) formed with a foot (116) being a free end portion of the anchor and comprising a contact surface configured for contacting tissue adjacent to the native heart valve, such that each of the plurality of anchors (114a-114d) has a foot angle from 0 to 45 degrees relative to the longitudinal axis (112), wherein the foot angle is defined as the angle between an axis running through the center of the feet (116) and the longitudinal axis (112) of the valve prosthesis (100), wherein each anchor (114a-114d) is expandable from a collapsed anchor configuration to an expanded anchor configuration; and wherein the contact surface of at least one of the plurality of spaced anchors (114a-114d) includes a hexagonal-shaped, diamond-like structure.

6. The method of claim 5 , wherein forming the plurality of anchors comprises forming at least one of the plurality of anchors such that the foot angle of the at least one of the plurality of anchors relative to the longitudinal axis is 0 degrees to load tissue of the annulus comprised of collagen and/or reticular fibers.

## Patentansprüche

1. Herzklappenprothese (100), die zum Einsetzen in eine native Herzklappe an einem nativen Herzklappenring (200) ausgeführt ist, wobei die Herzklappenprothese umfasst:
einen dehnbaren Rahmen (106), der ein proximales Ende (108) sowie ein distales Ende (110) und eine Längsachse (112) umfasst, die durch ihn hindurch verläuft, wobei der dehnbare Rahmen (106) so ausgeführt ist, dass er zur Einführung radial zusammengefaltet ist und sich bei Einsatz radial in eine entfaltete Konfiguration ausdehnt; sowie eine Vielzahl beabstandeter Anker (114a-114d), die sich von dem distalen Ende (110) des Rahmens (106) in Richtung des proximalen Endes (108) erstrecken, wobei jeder Anker (114a-114d) mit einem Fuß (116) versehen ist, der ein freier Endabschnitt des Ankers ist und eine Kontaktfläche umfasst, die so ausgeführt ist, dass sie mit an die native Herzklappe angrenzendem Gewebe in Kontakt kommt, und jeder Anker (114a-114d) von einer zusammengefalteten Anker-Konfiguration in eine entfaltete Anker-Konfiguration ausgedehnt werden kann; wobei jeder der Anker (114a-114d) einen Fuß-Winkel von 0° bis 45° relativ zu der Längsachse (112) einschließt, wobei der Fuß-Winkel als der Winkel zwischen einer Achse, die durch die Mitte der Füße (116) verläuft, und der Längsachse (112) der Herzklappenprothese (100) definiert ist; und wobei die Kontaktfläche wenigstens eines der Vielzahl beabstandeter Anker (114a-114d) eine sechseckig geformte rautenartige Struktur einschließt.

2. Herzklappenprothese nach Anspruch 1, wobei die Vielzahl beabstandeter Anker als Verankerungs-Füße ausgeführt sind.

3. Herzklappenprothese nach Anspruch 2, wobei die Prothese zwei vordere Anker und zwei hintere Anker enthält.

4. Herzklappenprothese nach einem der vorangehenden Ansprüche, wobei der Fuß-Winkel wenigstens eines der beabstandeten Anker relativ zu der Längsachse 0° beträgt, so dass der wenigstens eine der beabstandeten Anker so ausgeführt ist, dass er auf Gewebe des Rings drückt.

5. Verfahren zum Herstellen einer Herzklappenprothese (100), die zum Einsetzen in eine native Herzklappe an einem nativen Herzklappenring (200) ausgeführt ist, wobei das Verfahren umfasst:
Ausbilden eines dehnbaren Rahmens (106), der ein proximales Ende (108) sowie ein distales Ende (110) und eine Längsachse (112) umfasst, die durch ihn hindurch verläuft, wobei der dehnbare Rahmen (106) so ausgeführt ist, dass er zur Einführung radial zusammengefaltet ist und sich bei Einsatz radial in eine entfaltete Konfiguration ausdehnt; sowie Ausbilden einer Vielzahl beabstandeter Anker (114a-114d), die sich von dem distalen Ende (110) des Rahmens (106) in Richtung des proximalen Endes (108) erstrecken, wobei jeder Anker (114a-114d) mit einem Fuß (116) versehen ist, der ein freier Endabschnitt des Ankers ist und eine Kontaktfläche umfasst, die so ausgeführt ist, dass sie mit an die native Herzklappe angrenzendem Gewebe in Kontakt kommt, so dass jeder der Vielzahl von Ankern (114a-114d) einen Fuß-Winkel von 0° bis 45° relativ zu der Längsachse (112) hat, wobei der Fuß-Winkel als der Winkel zwischen einer Achse, die durch die Mitte der Füße (116) verläuft, und der Längsachse (112) der Herzklappenprothese (100) definiert ist, jeder Anker (114a-114d) von einer zusammengefalteten Anker-Konfiguration in eine entfaltete Anker-Konfiguration ausgedehnt werden kann; und wobei die Kontaktfläche wenigstens eines der Vielzahl beabstandeter Anker (114a-114d) eine sechseckig geformte rautenartige Struktur einschließt.

6. Verfahren nach Anspruch 5, wobei Ausbilden der Vielzahl von Ankern umfasst:
Ausbilden wenigstens eines der Vielzahl von Ankern so, dass der Fuß-Winkel des wenigstens einen der Vielzahl von Ankern relativ zu der Längsachse 0° beträgt und auf Gewebe des Rings gedrückt wird, das aus Kollagen und/oder retikulären Fasern besteht.

## Revendications

1. Prothèse de valvule (100) configurée pour être déployée au sein d'une valvule cardiaque native au niveau d'un anneau de valvule cardiaque native (200), la prothèse de valvule comprenant : un cadre dilatable (106) comprenant une extrémité proximale (108) et une extrémité distale (110) et un axe longitudinal (112) s'étendant à travers celui-ci, le cadre dilatable (106) étant configuré pour s'affaisser radialement pour mise en place et se dilater radialement lors du déploiement vers une configuration dilatée ; et une pluralité d'ancrages espacés (114a-114d) s'étendant depuis l'extrémité distale (110) du cadre (106) vers l'extrémité proximale (108), chaque ancrage (114a-114d) étant formé d'un pied (116) qui est une partie d'extrémité libre de l'ancrage et comprenant une surface de contact configurée pour entrer en contact avec un tissu adjacent à la valvule cardiaque native, et chaque ancrage (114a-114d) pouvant se dilater d'une configuration d'ancrage affaissée à une configuration d'ancrage dilatée ; dans laquelle chacun des ancrages (114a-114d) comporte un angle de pied de 0 à 45 degrés par rapport à l'axe longitudinal (112), dans laquelle l'angle de pied est défini comme l'angle entre un axe passant à travers le centre des pieds (116) et l'axe longitudinal (112) de la prothèse de valvule (100) ; et dans laquelle la surface de contact d'au moins un de la pluralité d'ancrages espacés (114a-114d) comporte une structure de forme hexagonale de type losange.

2. Prothèse de valvule selon la revendication 1 dans laquelle la pluralité d'ancrages espacés sont configurés sous forme de pieds d'ancrage.

3. Prothèse de valvule selon la revendication 2 dans laquelle la prothèse comporte deux ancrages antérieurs et deux ancrages postérieurs.

4. Prothèse de valvule selon l'une des revendications précédentes, dans laquelle l'angle de pied d'au moins un des ancrages espacés par rapport à l'axe longitudinal est de 0 degré, de sorte qu'au moins un des ancrages espacés est configuré pour charger un tissu de l'anneau.

5. Procédé de fabrication d'une prothèse de valvule (100) configurée pour être déployée au sein d'une valvule cardiaque native au niveau d'un anneau de valvule cardiaque native (200), le procédé comprenant : la formation d'un cadre dilatable (106) comprenant une extrémité proximale (108) et une extrémité distale (110) et un axe longitudinal (112) s'étendant à travers celui-ci, le cadre dilatable (106) étant configuré pour s'affaisser radialement pour mise en place et se dilater radialement lors du déploiement vers une configuration dilatée ; et la formation d'une pluralité d'ancrages (114a-114d) s'étendant depuis l'extrémité distale (110) du cadre (106) vers l'extrémité proximale (108), chaque ancrage (114a-114d) étant formé d'un pied (116) qui est une partie d'extrémité libre de l'ancrage et comprenant une surface de contact configurée pour entrer en contact avec un tissu adjacent à la valvule cardiaque native, de sorte que chacun de la pluralité d'ancrages (114a-114d) a un angle de pied de 0 à 45 degrés par rapport à l'axe longitudinal (112), dans lequel l'angle de pied est défini comme l'angle entre un axe passant à travers le centre des pieds (116) et l'axe longitudinal (112) de la prothèse de valvule (100), dans lequel chaque ancrage (114a-114d) peut se dilater d'une configuration d'ancrage affaissé à une configuration d'ancrage dilaté ; et dans lequel la surface de contact d'au moins un de la pluralité d'ancrages espacés (114a-114d) comporte une structure de forme hexagonale de type losange.

6. Procédé selon la revendication 5, dans lequel la formation de la pluralité d'ancrages comprend la formation d'au moins un de la pluralité d'ancrages de sorte que l'angle de pied du au moins un de la pluralité d'ancrages par rapport à l'axe longitudinal est de 0 degré pour charger un tissu de l'anneau composé de fibres de collagène et/ou réticulaires.
